# EUROPEAN PATENT SPECIFICATION

(11) **EP 4 001 251 B1**
(45) Date of publication and mention of the grant of the patent: **11.09.2024**
(21) Application number: 20841400.3
(22) Date of filing: 13.07.2020
(51) Int. Cl.: C07C 67/14, C07C 69/712

(54) **METHOD FOR PRODUCING BINAPHTHYLS**
VERFAHREN ZUR HERSTELLUNG VON BINAPHTHYLEN
PROCÉDÉ DE PRODUCTION DE BINAPHTYLS

(30) Priority: 17.07.2019 JP 2019132111
(43) Date of publication of application: 25.05.2022
(73) Proprietor: Honshu Chemical Industry Co., Ltd., Tokyo 103-0027 (JP)
(72) Inventor: SAKUMA, Daichi, Wakayama-shi, Wakayama 641-0007 (JP); SUTO, Takeru, Wakayama-shi, Wakayama 641-0007 (JP)
(74) Representative: Grünecker Patent- und Rechtsanwälte PartG mbB
(86) International application number: PCT/JP2020/027180
(87) International publication number: WO 2021/010363

(56) References cited:
- WO-A1-2012/120901
- WO-A1-2019/043060
- JP-A- 2018 059 074
- JP-A- 2019 210 277
- P. RAJAKUMAR, ET AL.: "Synthesis of novel carbazole based macrocyclic amides as potential antimicrobial agents", EUROPEAN JOURNAL OF MEDICINAL CHEMISTRY, vol. 44, no. 7, 31 July 2008 (2008-07-31), Elsevier Masson, Paris, FR, pages 3040 - 3045, XP026087813, ISSN: 0223-5234, DOI: 10.1016/j.ejmech.2008.07.031
- SHOCKRAVI, A. ET AL.: "Synthesis and characterization of novel thermally stable poly(ether-amide)s containing dinaphthosulfone units in the main chain", E-POLYMERS, vol. 11, no. 1, 044, 2011, pages 1 - 11, XP055788800, DOI: https://doi.org/10.1515/epoly. 2011.11.1 .491

## Description

### Technical Field

The present invention relates to a novel method for producing a binaphthyl.

### Background Art

Polyester resins and polyester carbonate resins produced using dicarboxylic acid components having a binaphthalene skeleton as polymerization components have excellent optical properties such as high refractive indices and low birefringence and have high levels of heat resistance, and thus have recently been expected to be materials for optical members such as optical disks, transparent conductive substrates, and optical filters. In particular, resins produced using 2,2'-bis(carboxymethoxy)-1,1'-binaphthyl or 2,2'-bis(ethoxycarbonylmethoxy)-1,1'-binaphthyl, which has a chemical structure represented by a chemical formula below, as a polymerization component have been attracting attention for their particularly excellent optical properties (see, for example, PTLs 1 to 3).

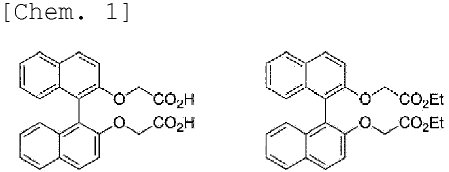

Known methods for producing 2,2'-bis(carboxymethoxy)-1,1'-binaphthyl or 2,2'-bis(ethoxycarbonylmethoxy)-1,1'-binaphthyl represented by the above chemical formula include a method in which 1,1'-binaphthalene-2,2'-diol and a halogenated acetic acid ester are reacted with each other and a method in which the diester obtained by the above method is hydrolyzed, as shown by a reaction formula below (see, for example, PTL 4).

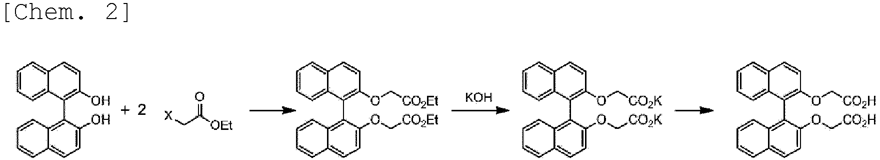

In the reaction between 1,1'-binaphthalene-2,2'-diol and a halogenated acetic acid ester in the above reactions, it is necessary, for example, to use a high-permittivity solvent as a reaction solvent or to use bromoacetic acid ester as a halogenated acetic acid ester in order to efficiently obtain the diester, which is a target compound. However, no studies have been made on methods for efficiently reacting, for example, a chloroacetic acid ester, which has low reactivity among halogenated acetic acid esters, in a low-permittivity solvent, and no reports have yet been made of production methods that achieve good reaction efficiency.

P. Rajakumar, et al., European Journal of Medicinal Chemistry, 2009, 44(7), 3040 to 3045 relates to carbazole macrocyclic amides as antimicrobial agents.

### Citation List

### Patent Literature

PTL 1: Japanese Unexamined Patent Application Publication No. 2018-002893
PTL 2: Japanese Unexamined Patent Application Publication No. 2018-002894
PTL 3: Japanese Unexamined Patent Application Publication No. 2018-002895
PTL 4: Japanese Unexamined Patent Application Publication No. 2008-024650

### Summary of Invention

### Technical Problem

The present invention has been made in view of the foregoing circumstances, and an object thereof is to provide a novel method for producing a binaphthyl, the method achieving good reaction efficiency and allowing an inorganic salt to be easily removed.

### Solution to Problem

To achieve the above object, the present inventors have conducted intensive studies and discovered that by performing a reaction in the presence of a specific solvent and an alkali metal iodide, good reaction efficiency is achieved and a target having a reduced inorganic salt content is efficiently obtained, thereby completing the present invention.

The present invention is as follows. 1. A method for producing a 2,2'-bis(alkoxycarbonylmethoxy)-1,1'-binaphthyl represented by formula (2) below, the method including reacting 1,1'-binaphthalene-2,2'-diol and a chloroacetic acid ester represented by formula (1) below with each other in a C5 to C8 aliphatic ketone solvent in the presence of an alkali metal iodide.
[Chem. 3]

ClCH₂COOR (1)

(In the formula, R represents a C1 to C8 alkyl group.) 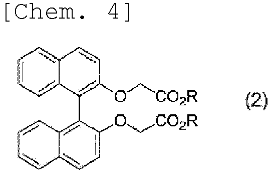 (In the formula, R represents a C1 to C8 alkyl group.) 2. The method for producing a 2,2'-bis(alkoxycarbonylmethoxy)-1,1'-binaphthyl according to 1, in which the alkali metal iodide is used in an amount of 3 mol% or more relative to an amount of 1,1'-binaphthalene-2,2'-diol.

### Advantageous Effects of Invention

According to the present invention, the reaction between 1,1'-binaphthalene-2,2'-diol and a halogenated acetic acid ester can be allowed to proceed with less by-products and at a sufficient reaction rate even in a low-permittivity ketone solvent such as methyl isobutyl ketone.

In addition, the use of a ketone solvent having low solubility in water, such as methyl isobutyl ketone, enables a water washing operation after the reaction and further enables a separation operation in a short time, thus allowing inorganic salts used in the reaction to be easily removed. This makes it possible to efficiently obtain a target having a reduced inorganic salt content.

That is, the provision of the production method of the present invention is very useful in industrial production of resin raw materials.

### Description of Embodiments

The present invention will be described below in detail.

A production method of the present invention is a method represented by the following reaction formula. 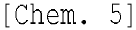 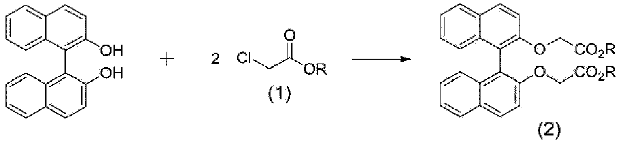 (In the formula, R represents a C1 to C8 alkyl group.)

### <Chloroacetic acid ester>

"R" in the formula of the chloroacetic acid ester used in the production method of the present invention is a C1 to C8 alkyl group, and specific examples include linear alkyl groups such as a methyl group, an ethyl group, and an n-propyl group, branched alkyl groups such as an i-propyl group and a t-butyl group, and cyclic alkyl groups such as a cyclohexyl group. In particular, C1 to C4 alkyl groups are preferred.

In the production method of the present invention, two or more chloroacetic acid esters having different alkyl ester moieties may be used in combination, but it is preferable to use a single chloroacetic acid ester in order to simplify the purification of the target 2,2'-bis(alkoxycarbonylmethoxy)-1,1'binaphthyl represented by formula (2) above.

The amount of chloroacetic acid ester used is not particularly limited as long as the molar ratio of the chloroacetic acid ester to 1,1'-binaphthalene-2,2'-diol is more than or equal to the theoretical value (2.0), but the chloroacetic acid ester is used typically in an amount of 2 mol or more, preferably in an amount of 2.1 to 3.0 mol, more preferably in an amount of 2.2 to 2.8 mol.

### <Reaction solvent>

The production method of the present invention is characterized in that the reaction is performed in a C5 to C8 aliphatic ketone solvent. Specific examples of C5 to C8 aliphatic ketone solvents include diethyl ketone (C5), methyl isobutyl ketone (C6), cyclohexanone (C6), methyl amyl ketone (C7), and 2-octanone (C8). These solvents may be used alone or in combination of two or more.

In particular, C6 to C8 aliphatic ketones are preferred. In terms of the structure of aliphatic ketones, linear or chain aliphatic ketones are preferred.

The amount of C5 to C8 aliphatic ketone solvent used is preferably in the range of 150 to 500 parts by weight, more preferably in the range of 200 to 300 parts by weight, relative to 100 parts by weight of 1,1'-binaphthalene-2,2'-diol.

### <Alkali metal iodide>

The production method of the present invention is characterized in that the reaction is performed in the presence of an alkali metal iodide. Specific examples of alkali metal iodides include potassium iodide, sodium iodide, cesium iodide, and lithium iodide. These may be used alone or in combination of two or more.

In particular, potassium iodide and sodium iodide are preferred.

The amount of alkali metal iodide used is preferably in the range of 1 to 25 mol%, more preferably in the range of 2 to 15 mol%, still more preferably in the range of 2.5 to 10 mol%, particularly preferably in the range of 3 to 5 mol%, relative to 1 mol of 1,1'-binaphthalene-2,2'-diol.

In the production method of the present invention, although the reaction is performed in a low-permittivity C5 to C8 aliphatic ketone solvent such as methyl isobutyl ketone, the presence of an alkali metal iodide can suppress the formation of by-products and allows the reaction to proceed at a sufficient reaction rate. Thus, the target 2,2'-bis(alkoxycarbonylmethoxy)-1,1'-binaphthyl represented by formula (2) above can be obtained with good reaction efficiency. This can shorten the reaction time, and thus this method is very useful in industrial production. In addition, the use of a C5 to C8 aliphatic ketone having low solubility in water, such as methyl isobutyl ketone, as a reaction solvent allows a water washing operation after the reaction to be easily performed without forming an emulsion. Accordingly, not only the time required for the post-reaction treatment can be shortened, but also the alkali metal iodide used in the reaction and an inorganic salt derived from a base that can be used in combination can be easily removed.

### <Reaction conditions>

The reaction temperature in the production method of the present invention is preferably in the range of 50°C to 150°C, more preferably in the range of 70°C to 130°C, still more preferably in the range of 90°C to 110°C. If the reaction temperature is high, the yield decreases due to, for example, hydrolysis of the target 2,2'-bis(alkoxycarbonylmethoxy)-1,1'binaphthyl. If the reaction temperature is low, the reaction rate will be slow, which is not preferred. The reaction is typically performed under normal pressure, but depending on the boiling point of the C5 to C8 aliphatic ketone solvent used, the reaction may be performed under pressure or reduced pressure so that the reaction temperature falls within the above range.

In the production method of the present invention, 1,1'-binaphthalene-2,2'-diol is preferably formed into a salt with a base before being reacted with a chloroacetic acid ester. Examples of bases suitable for use include alkali metal carbonates such as lithium carbonate, sodium carbonate, and potassium carbonate; alkali metal hydrogen carbonates such as sodium hydrogen carbonate and potassium hydrogen carbonate; alkali metal hydroxides such as lithium hydroxide, sodium hydroxide, and potassium hydroxide; and organic bases such as triethylamine and pyridine. In particular, sodium carbonate and potassium carbonate are preferred. The amount of base used is preferably 2.0 to 2.5 mol, more preferably 2.05 to 2.15 mol, relative to 1 mol of 1,1'-binaphthalene-2,2'-diol.

### <Reaction completion>

The endpoint of the reaction can be determined by liquid chromatography or gas chromatography analysis. The endpoint of the reaction is preferably defined as the time point at which unreacted 1,1'-binaphthalene-2,2'-diol has disappeared and the increase of the target is no longer observed. Although the reaction time varies depending on the reaction conditions such as reaction temperature, the reaction is typically completed in about 1 to 30 hours.

After completion of the reaction, water is added to the reaction solution, the mixture is stirred, and then the resultant is left to stand to separate an aqueous layer. This water washing operation is performed twice or more, whereby the inorganic salt in the reaction solution can be removed. The amount of water used in one water washing operation is preferably in the range of 150 to 600 parts by weight, more preferably in the range of 200 to 400 parts by weight, relative to 100 parts by weight of 1,1'-binaphthalene-2,2'-diol used, and the temperature in the operation is preferably in the range of 60°C to 100°C, more preferably in the range of 70°C to 90°C. The stirring may be performed in any manner as long as an oil layer is sufficiently brought into contact with an aqueous layer, and although the time required varies depending on the apparatus, about 30 minutes is usually sufficient. It is preferable to purify and isolate the target 2,2'-bis(alkoxycarbonylmethoxy)-1,1'-binaphthyl from the oil layer left after the water washing operation. For example, the target 2,2'-bis(alkoxycarbonylmethoxy)-1,1'-binaphthyl can be obtained by performing a post-treatment operation such as separation by crystallization, filtration, distillation, column chromatography, etc. after the water washing operation according to a conventional method. To further increase the purity, purification by distillation, recrystallization, or column chromatography may be performed according to a conventional method.

### EXAMPLES

The present invention will now be described more specifically with reference to Examples.

The method of analysis is as follows.

### <Method of analysis>

1. Determination of target concentration in reaction solution, reaction yield, and reaction endpoint
Measuring apparatus: high-performance liquid chromatography analyzer (manufactured by Shimadzu Corporation)
Pump: LC-20AD
Column oven: CTO-20A
Detector: SPD-20A
Column: HALO-C18
Oven temperature: 50°C
Flow rate: 0.7 ml/min
Mobile phase: (A) acetonitrile, (B) 0.1 vol% phosphoric acid aqueous solution
Gradient conditions: (A) % by volume (time from start of analysis)
   30% (0 min) -> 100% (12 min) -> 100% (15 min)
Detection wavelength: 280 nm

### <Example 1>

### • Production method of present invention

In a four-necked flask, 600 g of 1,1'-binaphthalene-2,2'-diol, 1500 g of methyl isobutyl ketone, 608.2 g of potassium carbonate, and 10.4 g of potassium iodide were placed, heated to 100°C, and stirred at this temperature for two hours. After the stirring, 600 g of methyl isobutyl ketone was distilled off under reduced pressure, after which a mixed solution of 565 g of ethyl chloroacetate and 5.1 g of N-methylpyrrolidone was prepared, and the mixed solution was then added dropwise while maintaining the temperature of the reaction solution at 90°C to 100°C. After further stirring for nine hours, the concentration (area normalization method) of the target in the reaction solution determined by high-performance liquid chromatography was 98.4%, and the reaction yield calculated using a calibration curve was 99.2%. Thereafter, 1500 g of water was added, and the mixture was heated to 80°C and then left to stand; the mixture separated into two transparent layers within five minutes. The residual potassium concentration in the oil layer left after removal of the aqueous layer was 0.19 wt%, and the potassium removal rate was 99.5%, confirming that a high inorganic salt-removing effect was produced. Water in an amount of 1500 g was further added to the oil layer, and the mixture was heated to 80°C and then left to stand; the mixture separated into two transparent layers within five minutes.

It was separately confirmed that in the case of a separation operation in which the mixture separates into two layers within five minutes on the experimental scale of Example 1 (total volume: 4 to 5 L), the mixture separates into two layers within one hour on a practical scale (total volume: 1000 to 2000 L), and the separation operation can be performed in a short time.

### <Comparative Example 1>

### • Production method without addition of alkali metal iodide

In a four-necked flask, 50 g of 1,1'-binaphthalene-2,2'-diol, 125 g of methyl isobutyl ketone, and 50.7 g of potassium carbonate were placed, heated to 100°C, and stirred at this temperature for two hours. After the stirring, 50 g of methyl isobutyl ketone was distilled off under reduced pressure, after which a mixed solution of 47.1 g of ethyl chloroacetate and 0.4 g of N-methylpyrrolidone was prepared, and the mixed solution was then added dropwise while maintaining the temperature of the reaction solution at 90°C to 100°C. After further stirring for nine hours, the concentration (area normalization method) of the target in the reaction solution determined by high-performance liquid chromatography was 81.8%, and the reaction yield calculated using the calibration curve was 77.5%. The reaction was further continued; the concentration (area normalization method) of the target in the reaction solution after 24-hour stirring was 83.1%, and the reaction yield calculated using the calibration curve was 76.4%.

### <Comparative Example 2>

### · Production method using reaction solvent other than C5 to C8 aliphatic ketone

In a four-necked flask, 30 g of 1,1'-binaphthalene-2,2'-diol, 90 g of acetonitrile, 30.4 g of potassium carbonate, and 0.52 g of potassium iodide were placed, heated to 70°C, and stirred at this temperature for one hour. A mixed solution of 28.2 g of ethyl chloroacetate and 0.3 g of N-methylpyrrolidone was prepared, and the mixed solution was then added dropwise while maintaining the temperature of the reaction solution at 70°C to 80°C. After further stirring for six hours, the concentration (area normalization method) of the target in the reaction solution determined by high-performance liquid chromatography was 99.3%, and the reaction yield calculated using the calibration curve was 99.6%. Thereafter, 75 g of water was added, and the mixture was heated to 70°C and then left to stand; the mixture separated into two transparent layers within five minutes. The residual potassium concentration in the oil layer left after removal of the aqueous layer was 1.22 wt%, and the potassium removal rate was 96.5%, confirming that the inorganic salt removal efficiency was worse than that of the production method of Example 1. Water in an amount of 75 g was further added to the oil layer, and the mixture was heated to 70°C and then left to stand; although the separation was poor due to emulsification of the oil layer, the mixture separated into two transparent layers after one hour.

Since it takes one hour for the mixture to separate into two layers on the experimental scale of Comparative Example 2 (total volume: 0.3 L), it will probably take a long time for a separation operation on a practical scale (total volume: 1000 to 2000 L). Thus, the production method of Comparative Example 2 was judged to be unemployable on a practical scale because of very poor production efficiency.

### <Comparative Example 3>

### · Production method of Comparative Example 2 without addition of alkali metal iodide

The reaction was performed in the same manner as in Comparative Example 2 without adding potassium iodide. After stirring for six hours, the concentration (area normalization method) of the target in the reaction solution determined by high-performance liquid chromatography was 84.4%, and the reaction yield calculated using the calibration curve was 83.0%. The reaction was further continued; the concentration (area normalization method) of the target in the reaction solution after 12-hour stirring was 99.0%, and the reaction yield calculated using the calibration curve was 99.2%.

Also in this production method, the separation operation required about one hour as in Comparative Example 2.

## Claims

1. A method for producing a 2,2'-bis(alkoxycarbonylmethoxy)-1,1'-binaphthyl represented by formula (2) below, the method comprising reacting 1,1'-binaphthalene-2,2'-diol and a chloroacetic acid ester represented by formula (1) below with each other in a C5 to C8 aliphatic ketone solvent in the presence of an alkali metal iodide,
[Chem. 1]
ClCH₂COOR (1)
where R represents a C1 to C8 alkyl group,
wherein the alkyl group is a linear alkyl group, branched alkyl group or cyclic alkyl group 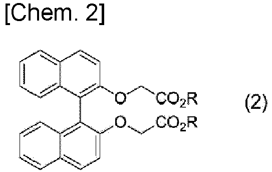
where R represents a C1 to C8 alkyl group.

2. The method for producing a 2,2'-bis(alkoxycarbonylmethoxy)-1,1'-binaphthyl according to Claim 1, wherein the alkali metal iodide is used in an amount of 3 mol% or more relative to an amount of 1,1'-binaphthalene-2,2'-diol.

## Patentansprüche

1. Verfahren zur Herstellung eines 2,2'-Bis(alkoxycarbonylmethoxy)-1,1'-binaphthyls, dargestellt durch die nachstehende Formel (2), wobei das Verfahren die Umsetzung von 1,1'-Binaphthyl-2,2'-diol und einem Chloressigsäureester, dargestellt durch die nachstehende Formel (1), miteinander in einem Lösungsmittel, das ein aliphatisches C5- bis C8-Keton ist, in Gegenwart eines Alkalimetalliodids umfasst,
[Chem. 1]
ClCH₂COOR (1)
wobei R eine C1- bis C8-Alkylgruppe darstellt,
wobei die Alkylgruppe eine lineare Alkylgruppe, eine verzweigte Alkylgruppe oder eine cyclische Alkylgruppe ist 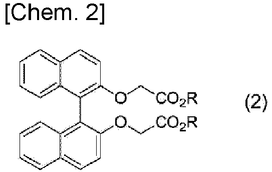
wobei R eine C1- bis C8-Alkylgruppe darstellt.

2. Verfahren zur Herstellung eines 2,2'-Bis(alkoxycarbonylmethoxy)-1,1'-binaphthyls nach Anspruch 1, wobei das Alkalimetalliodid in einer Menge von 3 Mol-% oder mehr, bezogen auf eine Menge von 1,1'-Binaphthyl-2,2'-diol, verwendet wird.

## Revendications

1. Procédé de production d'un 2,2'-bis(alcoxycarbonylméthoxy)-1,1'-binaphtyle représenté par la formule (2) ci-dessous, le procédé comprenant la réaction du 1,1'-binaphtalène-2,2'-diol et d'un ester d'acide chloroacétique représenté par la formule (1) ci-dessous l'un avec l'autre dans un solvant cétonique aliphatique en C5 à C8 en présence d'un iodure de métal alcalin,
[Substance chimique 1]
ClCH₂COOR (1)
où R représente un groupe alkyle en C1 à C8,
le groupe alkyle étant un groupe alkyle linéaire, un groupe alkyle ramifié ou un groupe alkyle cyclique 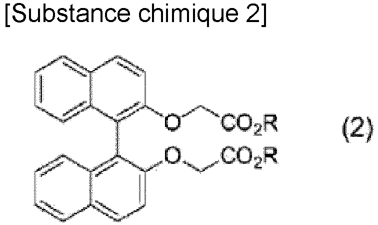
où R représente un groupe alkyle en C1 à C8.

2. Procédé de production d'un 2,2'-bis(alcoxycarbonylméthoxy)-1,1'-binaphtyle selon la revendication 1, dans lequel l'iodure de métal alcalin est employé à hauteur de 3 % en moles ou plus par rapport à une quantité de 1,1'-binaphtalène-2,2'-diol.
